# EUROPEAN PATENT APPLICATION

(11) **EP 1 652 524 A1**
(43) Date of publication of application: **03.05.2006**
(21) Application number: 04771333.4
(22) Date of filing: 06.08.2004
(51) Int. Cl.: A61K 31/4545, A61K 9/14, A61K 47/10, A61K 47/26, A61K 47/36, A61K 47/38, A61P 37/08

(54) **DRY SYRUP CONTAINING LORATADINE**

(30) Priority: 08.08.2003 JP 2003290052
(71) Applicant: SCHERING CORPORATION, Kenilworth, New Jersey 07033-0530 (US)
(72) Inventor: TOYODA, Toshitada, c/o Shionogi & Co. Ltd., Amagasaki-shi, Hyogo 6600813 (JP); MUGURUMA, Yoshitsugu, c/o Shionogi & Co. Ltd., Amagasaki-shi, Hyogo 6600813 (JP); TOMODA, Yoshitaka, c/o Shionogi & Co. Ltd., Amagasaki-shi, Hyogo 6600813 (JP)
(74) Representative: Hayes, Adrian Chetwynd
(86) International application number: PCT/JP2004/011333
(87) International publication number: WO 2005/013991

(57) **Abstract**

Dry syrup preparations comprising loratadine as a hydrophobic medicinal drug are provided. The loratadine dry syrup preparations can be produced using a cellulose material or an argininic acid salt together with sugar.

## Description

### TECHNICAL FIELD

The present invention is related to a new preparation of loratadine, specifically to a dry syrup preparation of loratadine.

### BACKGROUND ART

Loratadine is an active ingredient of a therapeutic agent for allergic diseases (a histamine H1 receptor antagonist) which is marketed under the trade name of "Claritin Tablet". The chemical name of loratadine is 4-(8-chloro-5,6-dihydro-1H-benzo[5,6]cyclohepta[1,2-b]pyridine-11-ylidene)-1-piperidinecarboxylic acid ethyl ester, and the compound was disclosed in Kokai publication JP57-35586 for the first time.

There are various types of pharmaceutical preparations such as tablet, powder, granule, capsules etc. Among them, a dry syrup preparation means "preparations which are dissolved or suspended before use" according to general rules for preparations in the Japanese pharmacopoeia. This is the preparation especially for patients like children who dislike a medicine or elderly persons having difficulty swallowing, and even the children and/or the elderly persons can easily take it. Furthermore, the preparation is handy, and weighing and preparing divided powder of this preparation are easy.

A liquid syrup preparation comprising an analog of loratadine, sucrose, sorbitol and water is disclosed in Kokai publication JP57-35586, but a dry syrup preparation is not described. In WO 02/05816, a study of preparations to improve bioavailability of loratadine is described but a dry syrup preparation of loratadine is not described.

In Kohyo Publication JP2000-508649, orally disintegrating preparations comprising (a) at least one active ingredient, (b) at least one extender such as sucrose etc., (c) at least one binder such as cellulose, and (d) at least one adjuvant such as talc, is disclosed. Specifically, fast-dissolving preparations for oral administration comprising potassium diclofenac, mannitol and polyvinylpyrrolidone is disclosed. But, a dry syrup preparation of loratadine is not described. In WO 01/26691, oral pharmaceutical composition with rapidly distintegrating property comprising an active ingredient soluble in water and sucrose is described. Specifically, a dry syrup preparation comprising faropenem sodium, sucrose and hydroxypropyl cellulose etc. is disclosed. But, a dry syrup preparation of loratadine is not described.

### DISCLOSURE OF INVENTION

In consideration of the merits of the dry syrup preparation comprising loratadine described above, the inventors studied to develop the said preparation. Loratadine is a hydrophobic compound, and when preparing a dry syrup preparation comprising such a hydrophobic compound, a surfactant and a defoaming agent are considered essential for blending the agent with water and defoaming the dispersion respectively. The inventors tried to find an alternative agent for the surfactant and/or the antifoam agent since the conventional agents were not enough to prepare the target dry syrup preparation.

The inventors continued their investigation and finally found that some kinds of binders usually used to granulate a mixed powder which are not easily granulated provide an excellent dry syrup preparation of loratadine, which yields a homogenous dispersion of loratadine with generating little foam when thrown into water, and completed the present invention.

Thus, the present invention relates to
(1) Dry syrup preparation comprising loratadine as an active ingredient, a binder that provides a homogenous dispersion upon addition of water at use, and a sugar; preferably, dry syrup preparation, wherein the said binder is a kind of celluloses or natural polymeric compounds; more preferably, dry syrup preparation, wherein either surfactant or deforming agent is not included; and dry syrup preparation showing the specific physical properties described in detail below and the method to provide the preparation;
(2) Dispersion in which loratadine is homogenously dispersed, comprising loratadine as an active ingredient, a binder that provides an homogenous dispersion upon addition of water at use, and a sugar; and specifically dispersion which is provided by throwing the dry syrup preparation of the present invention into water and stirring the mixture; and
(3) Method to improve the dispersibility of loratadine in water characterized by providing the dry syrup preparation combining loratadine with celluloses and/or a natural polymeric compound.

The dry syrup preparation, comprising loratadine as an active ingredient, a binder that upon addition of water at use, provides a homogenous dispersion such as celluloses, and a sugar, yields a homogenous dispersion of loratadine with generating little foam when thrown into water.

Also, the dry syrup preparation of the present invention provides a homogenous dispersion without bitter tastes. Accordingly, the dry syrup preparation of the present invention is easily taken by children who dislike a medicine or by elderly persons having difficulty swallowing. Furthermore, the preparation is handy, and weighing and preparing divided powder of this preparation are easy.

### BEST MODE FOR CARRYING OUT THE INVENTION

### (1)Dry syrup preparation

In the first aspect, the present invention provides a dry syrup preparation comprising loratadine as an active ingredient, a binder that upon addition of water at use, provides a homogenous dispersion, and a sugar.

The dry syrup preparation of the invention turns into a homogenous dispersion upon addition of water at use. The homogenous dispersion means a preparation having the physical properties shown below;
(i) sedimantation is observed within one minute after 5g of the preparation is thrown into 100ml of water;
(ii) the mixture is turned cloudy and dispersed after 5g of the preparation is thrown into 100ml of water, upset and turned back and left at rest;
(iii) the mixture is turned cloudy and dispersed after 5g of the preparation is thrown into 100ml of water, upset and turned back, and left for a day, then, upset and turned back again and left at rest;
(iv) no suspended substance is observed within one minute after evaluation of the dispersibility; and/or
(v) bubbles are decreased within one minute after evaluation of the dispersibility.

These properties above are termed (i) sedimantation property, (ii) dispersibility, (iii) re-dispersibility, (iv) presence or absence of supernatant, and (v) defoaming property, respectively. In this specification, these properties may be collectively termed "homogenous dispersibility" and details of the physical properties are explained in Test Example 1 below.

Loratadine, which is an active ingredient of the dry syrup preparation of the present invention, is easily prepared from the known starting substance using the method described in Kokai publication JP57-35586.

"The binder that provides a homogenous dispersion upon addition of water at use" comprised in the dry syrup preparation of the invention means the binder that provides a preparation having the physical properties described above, and includes celluloses, natural polymeric compounds, starch and its derivatives, and synthetic polymeric compounds etc.
Specifically, celluloses include hydroxypropyl cellulose, hydroxypropylmethyl cellulose, methyl cellulose, carmelose sodium, crystalline cellulose carmelose sodium, crystalline cellulose, powder cellulose, hydroxypropylmethyl cellulose phthalate, hydroxypropylmethyl cellulose acetate succinate, carboxymethylethyl cellulose, and hydroxyethyl cellulose etc.
Natural polymeric compounds include alginic acid and its salt, guar gum, tragacanth, tragacanth powder, carrageenan, gum arabic, powdered gum arabic, agar, powdered agar, white shellac, xanthan gum, and gelatine etc. Starch and its derivatives include wheat starch, rice starch, corn starch, potato starch, dextrin, pregelatinized starch, partially pregelatinized starch, hydroxypropyl starch, and pullulan etc.
Synthetic polymeric compounds include polyvinylpyrrolidone K25, polyvinylpyrrolidone K30, polyvinylpyrrolidone K90, aminoalkyl methacrylate copolymer E, aminoalkyl methacrylate copolymer RS, methacrylic acid copolymer L, methacrylic acid copolymer S, methacrylic acid copolymer LD, carboxyvinyl polymer, polyvinylacetal diethylaminoacetate and polyvinyl alcohol etc.
At least one of the binders used in the present invention is selected from the group exemplified above. Among them, hydroxypropyl cellulose in celluloses or alginate in natural polymeric compounds is preferable, and especially hydroxypropyl cellulose is preferable. There are three kinds of hydroxypropyl cellulose, the viscosities of which in 2% aqueous solution at 20°C are below 3.0 mPa s, 3.0-5.9 mPa s and 6.0-10.0 mPa s respectively. In the present invention, hydroxypropyl cellulose having the viscosity below 3.0 mPa s is preferable.

Sugars contained in the dry syrup preparation of the present invention include saccharide and sugar alcohol, as exemplified by at least one kind of compound selected from the group of sucrose, glucose, mannitol, powdered hydrogenated maltose syrup, maltitol, erythritol, sorbitol, maltose, lactose, starch and starch derivative, mannose, sorbose, xylose, trehalose, fructose, dextran, pullulan, dextrin, cyclodextrin, soluble starch, hydroxyethyl starch, sodium carboxymethyl cellulose, inositol, dulcitol, xylitol, arabitol, raffinose, lactitol, and palatinitt(trade name) etc. Among them, sucrose is preferable. Additionally, the sugars may include natural sweeteners or synthetic sweeteners of non-sugar, such as aspartame, glycyrrhizinic acid and its salt, saccharin and its salt, stevia and its salt, sucralose, and acesulfame potassium etc.

Pharmaceutically acceptable additives other than those described above may be included in the dry syrup preparation of the present invention. Lubricant, suspending agent, pH-adjusting agent, preservative, and/or flavor are included in the additives.

Lubricants include filler, absorbent, or plasticizer, as exemplified by at least one kind of compound selected from silicon dioxide hydrate, light anhydrous silicic acid, fatty acid ester of sucrose, and magnesium stearate etc. Suspending agents include disperser, viscosity-increasing agent, or disintegrant, and the binder described above may be used for example.
pH-adjusting agents include acids, bases or buffers, as exemplified by at least one kind of compound selected from hydrochloric acid, diluted hydrochloric acid, adipic acid and its salt, citric acid and its salt, gluconic acid and its salt, succinic acid and its salt, ascorbic acid and its salt, glacial acetic acid and its salt, acetic acid and its salt, tartaric acid and its salt, fumaric acid and its salt, maleic acid and its salt, lactic acid and its salt, malic acid and its salt, phosphoric acid and its salt, glycine, sodium biocarbonate, sodium carbonate, sodium hydroxide, and magnesium hydroxide etc.

Preservatives include stabilizers, as exemplified by at least one kind of compound selected from e.g., benzoic acid and its salt, edetic acid and its salt, salicylic acid and its salt, dibutylhydroxytoluene, sorbic acid and its salt, sodium dehydroacetate, and paraoxybenzoic acid and its salt etc. Flavors include flavoring agents, as exemplified by at least one kind of compound selected from orange essence, orange oil, caramel, camphor, cinnamon oil, spearmint oil, strawberry essence, chocolate essence, cherry flavor, orange peel oil, pine oil, mint oil, vanilla flavor, bitter essence, fruit flavor, peppermint essence, mixed flavor, mint flavor, menthol, lemon powder, lemon oil, and rose oil etc.

The composition of the dry syrup preparation of the present invention is for example, 0.01 - 50 (w/w) % of loratadine, 0.5 - 20.0 (w/w) % of a binder, and 20.0 - 99.49 (w/w) % of a sugar; preferably, 0.1 - 10 (w/w) % of loratadine, 0.5 - 10.0 (w/w) % of a binder, and 50.0 - 99.4 (w/w) % of a sugar; more preferably, 0.5 - 5.0 (w/w) % of loratadine, 0.5 - 5.0 (w/w) % of a binder, and 80.0 - 99.0 (w/w) % of a sugar; and most preferably, 0.5 - 3.0 (w/w) % of loratadine, 0.5 - 1.0 (w/w) % of a binder, and 90.0 - 99.0 (w/w) % of a sugar. The preferable ingredients of the present dry syrup preparation is the combination of loratadine, hydroxypropyl cellulose as a binder, and sucrose as a sugar. The dry syrup preparation comprised of 0.5 - 3.0 (w/w) % of loratadine, 0.5 -1.0 (w/w) % of hydroxypropyl cellulose, 0.25 - 0.75 (w/w) % of silicon dioxide hydrate and 90.0 - 98.75 (w/w) % of sucrose is mostly preferable.

It was found that a suitable dry syrup preparation was not obtained if a surfactant or a defoaming agent is additionally included together with the binder specified above when preparing the dry syrup preparation of loratadine. Accordingly, either a surfactant or a defoaming agent is not included in a preferred embodiment of the present invention. The surfactant which is excluded in the preferred embodiment of the present invention is fatty acid ester of sucrose, fatty acid ester of sorbitan, polyoxyethylene hydrogenated castor oil, polysorbate 80, or sodium lauryl sulfate. The defoaming agent which is excluded in the preferred embodiment of the present invention is silicon resin, silicon resin emulsion, silicon defoaming agent, silicon oil, fatty acid ester of sucrose, fatty acid ester of glycerin, dimethylpolysiloxane, dimethylpolysiloxane silicon dioxide mixture, polyoxyl stearate 40, fatty acid ester of sorbitan, sorbitan torioleate, or polysorbate 80.

With respect to a particle size of dry syrup preparation there are no clear-cut rules, but in general the size is in the range of powder, granule and fine granule preparations in Japanese Pharmacopoeia.

The dry syrup preparation of the present invention is produced using a method for producing a preparation of powders, granules, or fine granules, as exemplified by mixing-granulating method, extrusion-granulating method, fluidized bed-granulating method, chopping granulating method, spray-granulating method, and crushing granulating method.

A brief explanation of the mixing granulating method is as follows; a given amount of loratadine and sugar are passed through a sieve having opening of 425µm and the resulting powder is mixed in a mixing granulator. Next, a given amount of a binder is added and the granulating is continued for a given amount of time. Then, it is dried in a suitable fluidized bed granulator and granulated using a basket with opening of 1038µm. If necessary, fine powders may be eliminated using a classifier having wire gauze with opening of 154µm. The objective dry syrup preparation may be obtained by adding suitable additives and mixing with them. A part or all of the additives may be sieved with loratadine and the sugars at the same time.

The extrusion-granulation method may be carried out in the same manner as the mixing granulation method except using an extrusion granulator after mixing the starting materials containing additives and granulating them. As the extrusion granulator, e.g., Domegran DGL1 Model (dice diameter: 0.5mm, manufactured by Fuji Paudal Co.Ltd.) or Cylindrical Granulator(dice diameter: 0.53mm, manufactured by Yamada Iron Works Co.Ltd.) may be used.

The fluidized bed-granulating method may be carried out in the same manner as the mixing granulation method except using a fluidized bed-granulator in place of the mixing granulator used in the method. As the fluidized bed-granulator, Granulating Dryer WSG-5 Model (Manufactured by Okawara MFG Co. Ltd.) may be used for example.

In the other aspect, the present invention provides a method for producing the dry syrup preparation characterized by mixing loratadine as an active ingredient, a sugar and an aqueous solution of binder that provides a homogenious dispersion upon addition of water at use, granulating and drying them.

### (2)Dispersion

In the other aspect, the present invention provides a dispersion in which loratadine is homogenously dispersed, comprising loratadine as an active ingredient, a binder that provides an homogenous dispersion upon addition of water at use, and a sugar.

The dispersion of the present invention is provided by throwing the dry syrup preparation of the invention into a suitable amount of water and stirring the mixture.

### (3)Method for improving the dispersibility of loratadine in water

In the other aspect, the present invention provides a method for improving the dispersibility of loratadine in water characterized by providing the dry syrup preparation combining loratadine with celluloses and/or natural polymeric compounds. Details are the same as described above. The term, "to improve the dispersibility in water" here means to prepare a homogenous dispersion as defined in the specification, comprising water and loratadine, which is a hydrophobic compound.

### EXAMPLE

The present invention is explained in more details below by working examples and test examples, but not limited to these examples.

### Example 1

### Preparing the dry syrup preparation using the mixing granulating method

A dry syrup preparation having the following composition (w/w%) and a control preparation are prepared.

**[Table 1] composition: (w/w%)**

| | Example 1 | Control 1 |
|---|---|---|
| loratadine | 1.0 | 1.0 |
| sucrose | 97.9 | 98.5 |
| hydroxypropyl cellulose | 0.6 | - |
| polyvinylpyrrolidone | - | - |
| dextrin | - | - |
| Silicon dioxide hydrate | 0.5 | 0.5 |
| Total | 100.0 | 100.0 |

**[Table 2] unit: (w/w%)**

| | Control 2 | Control 3 | Control 4 | Control 5 |
|---|---|---|---|---|
| loratadine | 1.0 | 1.0 | 1.0 | 1.0 |
| sucrose | 97.5 | 98.0 | 97.0 | 94.0 |
| Polysorbate 80 (surfactant) | 1.0 | - | 1.0 | 1.0 |
| silicon resin (defoaming agent) | - | 0.5 | 0.5 | 0.5 |
| hydroxypropyl cellulose | - | - | - | 3.0 |
| silicon dioxide hydrate | 0.5 | 0.5 | 0.5 | 0.5 |
| Total | 100.0 | 100.0 | 100.0 | 100.0 |

According to the composition table above, a necessary amount of each material is calculated based on the manufacturing scale. Sucrose, hydroxypropyl cellulose(HPC-SSL, the viscosity of 2% aquous solution at 20°C <3.0 mPa s) and silicon dioxide hydrate were used as a sugar, binder and lubricant, respectively.

In a case of Example 1, 20g of loratadine and 1,958g of sucrose were weighed and passed through a sieve with opening of 425µm, and the resulting powder was mixed in the mixing granulator (High speed mixer Model 10, manufactured by FUKAE Powtec/aditator:300rpm, chopper:2500rpm) for one minute. Then, 120g of 10(w/w)% aquous solution of hyrdoxypropyl cellulose was added and granulated for 3 minutes, dried in the fluidized bed-granulator (Granulating Dryer WSG-5 Model, manufactured by Okawara MFG Co.Ltd., blast temperature:55°C) and drying was terminated when the product temperature reached 45°C. After that, the product was granulated with the granulator (Powermill, P-3 Model) using a basket with opening of 1038µm. If necessary, fine powders may be eliminated using a classifier (Vibration Separator, TMC-50-2S Model, manufactured by Tokuju Corporation)having wire gauze with opening of 154µm. 9g of silicon dioxide hydrate, which was a necessary amount based on the calculation according to the composition table above, was added to the resulting granulation (1,791g) and mixed in the mixer (Mixer 8LV Model) to give the dry syrup preparation.

In a case of Control 1, necessary amounts of loratadine and sucrose are weighed and passed through a sieve with opening of 425µm, and the resulting powder was treated in the similar way as Example 1 except that 120g of purified water was added in place of hydroxypropyl cellulose when granulated. Control preparations 2-5 are also produced in the similar way as Example 1, but polysorbate 80 (surfactant) or silicon resin (defoaming agent) are also included in these preparations.

### Preparing the dry syrup preparation using the extrusion-granulating method

According to the composition table of Table 1 above, a necessary amount of each material is calculated based on the manufacturing scale. In a case of Example 1, 20g of loratadine and 1,958g of sucrose were weighed and passed through a sieve with opening of 425µm, and the resulting powder was mixed in the mixing granulator (High speed mixer Model 10, manufactured by FUKAE Powtec/aditator:300rpm, chopper:2500rpm) for one minute. Then, 120g of 10(w/w)% aqueous solution of hyrdoxypropyl cellulose was added and granulated for 3 minutes. After then, it was granulated in the extrusion granulator (Cylindrical Granulator, manufactured by Yamada Iron Works Co.Ltd. dice diameter: 0.53mm), dried in the fluidized bed-granulator (Granulating Dryer WSG-5 Model, manufactured by Okawara MFG Co.Ltd., blast temperature:55°C) and drying was terminated when the product temperature reached 45°C. After that, the product was granulated with the granulator (Powermill, P-3 Model) using a basket with opening of 1038µm. If necessary, fine powders may be eliminated using a classifier (Vibration Separator, TMC-50-2S Model, manufactured by Tokuju Corporation)having wire gauze with opening of 154µm. 9g of silicon dioxide hydrate, which was a necessary amount based on the calculation according to the composition table above, was added to the resulting granule (1,791g) and mixed in the mixer (Mixer 8LV Model) to give the dry syrup preparation.

In a case of Control 1, necessary amounts of loratadine and sucrose are weighed and passed through a sieve with opening of 425µm, and the resulting powder was treated in the similar way as Example 1 except that 120g of purified water was added in place of hydroxypropyl cellulose when granulated. Control preparations 2-5 are also produced in the similar way as Example 1, but polysorbate 80 (surfactant) or silicon resin (defoaming agent) are also included in these preparations.

### Preparing the dry syrup preparation using the fluidized bed granulating method

According to the composition table of Table 1 above, a necessary amount of each material is calculated based on the manufacturing scale. In a case of Example 1, 50g of loratadine and 4,895g of sucrose were weighed and passed through a sieve with opening of 425µm, and the resulting powder was mixed in the fluidized bed granulator (Granulating Dryer WSG-5 Model, manufactured by Okawara MFG Co.Ltd.) for five minutes. Then, 1,500g of 2(w/w)% aquous solution of hyrdoxypropyl cellulose was sprayed (spray speed:30g/min, spray pressure:0.15MPa) and the it was dried until the product temperature reached 45°C. After that, the product was granulated with the granulator (Powermill, P-3 Model) using a basket with opening of 1038µm. If necessary, fine powders may be eliminated using a classifier (Vibration Separator, TMC-50-2S Model, manufactured by Tokuju Corporation)having wire gauze with opening of 154µm. 24g of silicon dioxide hydrate, which was a necessary amount based on the calculation according to the composition table above, was added to the resulting granulation (e.g.,4,776g) and mixed in the mixer (Mixer 22LV Model) to give the dry syrup preparation.

In a case of Control 1, necessary amounts of loratadine and sucrose are weighed and passed through a sieve with opening of 425µm, and the resulting powder was treated in the similar way as Example 1 except that 1,500g of purified water was used as a spray solution of granulation. Control preparations 2-5 are also produced in the similar way as Example 1, but polysorbate 80 (surfactant) or silicon resin (defoaming agent) are also included in these preparations.

### Test Example 1

### Evaluation of preparations

The preparations of example and control described above were evaluated from the five aspects of (i) sedimantation property, (ii) dispersibility, (iii) re-dispersibility, (iv) presence or absence of supernatant, and (v) defoaming property.
(i) Method for evaluating sedimentation property
5g of the dry syrup preparation was thrown into 100 mL of water in a measuring cylinder with a stopper, and the time required for the preparation to sediment below the water surface was measured. It was judged "YES" if it sedimented within one minute, and judged "NO" if it did not.
(ii) Method for evaluating dispersibility
5g of the dry syrup preparation was thrown into 100 mL of water in a measuring cylinder with a stopper. After putting a stopper in the cylinder quickly, the top of the measuring cylinder was held with one hand and the bottom was held with the other hand, and it was turned upside down by rotating 180-degree around the bottom and turned back five times at a speed of two seconds for the upset and return, and left at rest. According to visual observation, it was judged Excellent, if the mixture was clouded in whole. If the mixture was not clouded, the cylinder was upset and turned back fifteen times more and left at rest. According to visual observation, it was judged Yes, if the mixture was clouded in whole, and judged No if it was not.
(iii) Method for evaluating re-dispersibility
The centrifuge tubes used in the test of evaluating dispersibility were left at room temperature for a day. After then, they were judged in the same method as evaluating dispersibility described above.
(iv) Presence or absence of supernatant
Just after evaluating dispersibility, stoppers of the centrifuge tubes were taken off and the contents were visually observed from above. With respect to the presence or absence of supernatant, it was judged "YES" if no suspended substance is observed within one minute, and judged "NO", if observed.
(v) Method for evaluating defoaming property
With respect to the defoaming property, it was judged "YES" if bubbles are decreased and the water surface was observed within one minute after evaluation of the dispersibility, and judged "No" if not. In the present preparation, almost of bubbles disappeared within one minute even if they were about 1 cm in height above the surface just after the shaking. But, bubbles not always disappear and sometimes small bubbles may be left. Accordingly, the term, "bubbles are decreased within one minute" means that the surface is observed within one minute, includes cases that small bubbles are left on the surface, and it is preferable that all the bubbles disappeared.

**[Table 3]**

| | Example 1 | Control 1 | Control 2 | Control 3 | Control 4 |
|---|---|---|---|---|---|
| sedimentation property | YES | YES | YES | YES | YES |
| dispersibility | YES | YES | YES | YES | YES |
| re-dispersibility (after a day) | YES | YES | YES | YES | YES |
| presence/absence of supernatant | YES | NO | NO | NO | NO |
| defoaming property | YES | NO | NO | YES | YES |

The preparation of Control 1 does not include hydroxypropyl cellulose, and the preparations of Control 2-5 additionally include a surfactant and a defoaming agent; in these points the preparation of Control 1-5 are different from the present invention respectively. Thus, it is shown that the dry syrup preparation, wherein hydroxypropyl cellulose is used as a single binder, and either a surfactant or a defoaming agent is not included, may improve the dispersibility of loratadine in water.

### Examples 2-7

### Preparing the dry syrup preparations containing various sugars.

According to the procedure described in Example 1, preparations of the compositions shown in the Table 4 below were prepared.

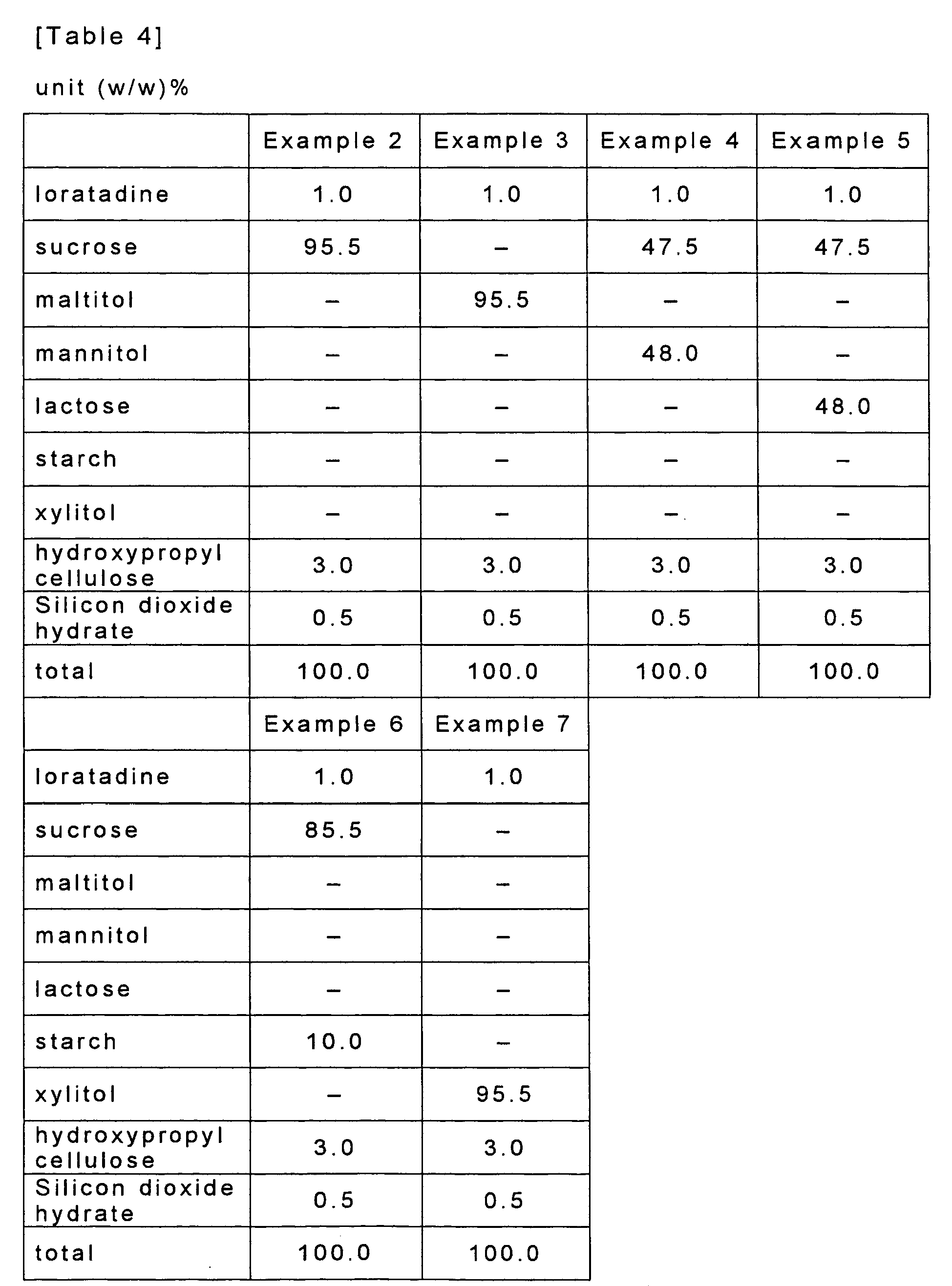

### Test Example 2

### Evaluation of the preparations

According to the methods described in Example 1, the homogenous dispersibilities of the preparations in Example 2-7 were evaluated. The result is shown in Table 5 below.

**[Table 5]**

| | Example 2 | Example 3 | Example 4 | Example 5 | Example 6 | Example 7 |
|---|---|---|---|---|---|---|
| sedimentation property | YES | YES | YES | YES | YES | YES |
| dispersibility | YES | YES | YES | YES | YES | YES |
| re-dispersibility (after a day) | YES | YES | YES | YES | YES | YES |
| presence/ absence of supernatant | YES | YES | YES | YES | YES | YES |
| defoaming property | YES | YES | YES | YES | YES | YES |

The results showed that maltitol, mannitol, lactose, and/or starch may be useful to improve the homogenous dispersibility in the same way.

### Examples 8-11

### Preparing the dry syrup preparations containing various suspending agents

According to the procedure described in Example 1, preparations of the compositions shown in the Table 6 below were prepared.

**[Table 6] unit (w/w) %**

| | Example 8 | Example 9 | Example 10 | Example 11 |
|---|---|---|---|---|
| loratadine | 1.0 | 1.0 | 1.0 | 1.0 |
| sucrose | 92.5 | 94.5 | 92.5 | 94.5 |
| crystalline cellulose carmellose sodium | 3.0 | - | - | - |
| Sodium alginate | - | 1.0 | - | - |
| Methyl cellulose | - | - | 3.0 | - |
| carmellose sodium | - | - | - | 1.0 |
| hydroxypropyl cellulose | 3.0 | 3.0 | 3.0 | 3.0 |
| Silicon dioxide hydrate | 0.5 | 0.5 | 0.5 | 0.5 |
| total | 100.0 | 100.0 | 100.0 | 100.0 |

### Test Example 3

### Evaluation of the preparations

According to the methods described in Example 1, the homogenous dispersibilities of the preparations in Example 8-11 were evaluated. The result is shown in Table 7 below.

**[Table 7]**

| | Example 8 | Example 9 | Example 10 | Example 11 |
|---|---|---|---|---|
| sedimentation property | YES | YES | YES | YES |
| dispersibility | YES | YES | YES | EX |
| re-dispersibility (after a day) | EX | EX | EX | EX |
| presence/absence of supernatant | YES | YES | YES | YES |
| defoaming property | YES | YES | YES | YES |

| | | | | |
|---|---|---|---|---|
| EX: excellent | | | | |

The result showed that the combination of hydroxypropyl cellulose with crystalline cellulose carmellose sodium, sodium alginate, methyl cellulose, and carmellose sodium might further improve the homogenous dispersibility.

### INDUSTRIAL APPLICABILITY

The dry syrup preparation of the present invention provides a homogenous dispersion without bitter taste, and the preparation is easily taken by children who dislike a medicine or by elderly persons having difficulty swallowing. Furthermore, it is handy, and weighing and preparing divided powder of this preparation are easy. Accordingly the present invention provides a useful medicine.

## Claims

1. Dry syrup preparation comprising loratadine as an active ingredient, a binder that provides a uniform dispersion upon addition of water at use, and a sugar.

2. Dry syrup preparation according to claim 1, wherein the binder is selected from celluloses.

3. Dry syrup preparation according to claim 2, wherein the celluloses are comprised of one or more selected from the group of hydroxypropyl cellulose, hydroxypropylmethyl cellulose, methyl cellulose, carmellose sodium, crystalline cellulose carmellose sodium, crystalline cellulose, powdered cellulose, hydroxypropylmethyl cellulose phthalate, hydroxypropylmethyl cellulose acetate succinate, carboxymethylethylcellulose and hydroxyethylcellulose.

4. Dry syrup preparation according to claim 3, wherein the celluloses are hydroxypropyl cellulose.

5. Dry syrup preparation according to claim 4, wherein the viscosity of 2% aqueous solution of the hydroxypropyl cellulose is below 3.0 mPa s at 20°C.

6. Dry syrup preparation according to claim 1, wherein the binder is a natural polymeric compound.

7. Dry syrup preparation according to claim 6, wherein the natural polymeric compound is alginate.

8. Dry syrup preparation according to any of claim 1-7, wherein the sugar is saccharide or sugar alcohol.

9. Dry syrup preparation according to claim 8, wherein the sugar is one or more selected from the group of sucrose, maltitol, mannitol, lactose and xylitol.

10. Dry syrup preparation according to claim 9, wherein the sugar is sucrose.

11. Dry syrup preparation according to any of claim 1-10, wherein no surfactant or defoaming agent is included.

12. Dry syrup preparation according to any of claim 1-11, having physical properties described below;
(i) sedimantation is observed within one minute after 5g of the preparation is thrown into 100ml of water;
(ii) the mixture is turned cloudy and dispersed after 5g of the preparation is thrown into 100ml of water, upset and turned back and left at rest;
(iii) the mixture is turned cloudy and dispersed after 5g of the preparation is thrown into 100ml of water, upset and turned back, and left for a day, then, upset and turned back again and left at rest;
(iv) no suspended substance is observed within one minute after evaluation of the dispersibility; and/or
(v) bubbles are decreased within one minute after evaluation of the dispersibility.

13. Method to provide dry syrup preparation **characterized in** mixing loratadine as an active ingredient, a sugar and an aqueous solution of binder that provides a uniform dispersion upon addition of water at use, granulating and drying them.

14. Dispersion in which loratadine is uniformly dispersed, comprising loratadine as an active ingredient, a binder that provides an uniform dispersion upon addition of water at use, and a sugar.

15. Dispersion according to claim 14, which is provided by throwing the dry syrup preparation of any of claim 1-12 into water and stirring the mixture.

16. Dispersion according to claim 14, having the physical properties described below;
(i) sedimentation is observed within one minute after 5g of the preparation is thrown into 100ml of water;
(ii) the mixture is turned cloudy and dispersed after 5g of the preparation is thrown into 100ml of water, upset and turned back and left at rest;
(iii) the mixture is turned cloudy and dispersed after 5g of the preparation is thrown into 100ml of water, upset and turned back, and left for a day, then, upset and turned back again and left at rest;
(iv) no suspended substance is observed within one minute after evaluation of the dispersibility; and/or
(v) bubbles are decreased within one minute after evaluation of the dispersibility.

17. Method to improve the dispersibility of loratadine in water **characterized in** providing the dry syrup preparation combining loratadine with celluloses and/or a natural polymeric compound.

18. Dry syrup preparation comprising 0.5 - 3.0 (w/w)% of loratadine, 0.5 - 1.0 (w/w)% of hydroxypropylcellulose, 0.25 - 0.75 (w/w)% of silicon dioxide hydrate and 90.0 - 98.75 (w/w)% of sucrose.
